Europäisches Patentamt

European Patent Office

Office européen des brevets

(19)

(11) Numéro de publication: **0 010 470**
**B1**

(12) **FASCICULE DE BREVET EUROPEEN**

(45) Date de publication du fascicule de brevet: **04.11.81**

(51) Int. Cl.³: **C 07 C 79/37, C 07 C 76/00**

(21) Numéro de dépôt: **79400688.2**

(22) Date de dépôt: **27.09.79**

(54) **Procédé pour la préparation des dérivés nitro-5 et 6 de la tétrahydro-1,2,3,4 anthraquinone à partir de l'hexahydro-1,2,3,4,4a,9a anthracène-dione-9,10.**

(30) Priorité: **17.10.78 FR 7829507**

(43) Date de publication de la demande:
**30.04.80 Bulletin 80/9**

(45) Mention de la délivrance du brevet:
**04.11.81 Bulletin 81/44**

(84) Etats Contractants Désignés:
**BE CH DE FR GB IT NL**

(56) Documents cités:
**FR - A - 658 972
FR - A - 1 224 885**

(73) Titulaire: **P C U K PRODUITS CHIMIQUES UGINE KUHLMANN**
**Service Propriété Industrielle Tour Manhattan**
**F-92087 PARIS LA DEFENSE 2 Cédex 21 (FR)**

(72) Inventeur: **Delavarenne, Serge**
**2, rue des Alouettes**
**F-69340 Francheville le Haut (FR)**
Inventeur: **Tellier, Pierre**
**106, avenue de la Libération**
**F-69110 Sainte Foy Les Lyon (FR)**

(74) Mandataire: **Houssin, Jean et al,**
**PRODUITS CHIMIQUES UGINE KUHLMANN**
**Service Propriété Industrielle Tour Manhattan**
**Cedex 21**
**F-92087 Paris La Defense 2 (FR)**

Courier Press, Leamington Spa, England.

# 0010470

Procédé pour la préparation des dérivés nitro-5 et 6 de la tétrahydro-1,2,3,4 anthraquinone à partir de l'hexahydro-1,2,3,4,4a,9a anthracène-dione-9,10.

La présente invention concerne la préparation des dérivés mononitrés en position 5 ou 6 de la tétrahydro-1,2,3,4 anthraquinone par nitration de l'hexahydro-1,2,3,4,4a,9a anthracène-dione-9,10. Ces dérivés sont des intermédiaires qui peuvent servir à la préparation de dérivés mononitrés en position $\alpha$ et $\beta$ de l'anthraquinone.

Les dérivés mononitrés en position $\alpha$ et $\beta$ de l'anthraquinone sont d'importants intermédiaires dans la fabrication des colorants. On sait que l'$\alpha$-nitro-anthraquinone peut être obtenue par nitration de l'anthraquinone. Toutefois cette réaction conduit à la formation inévitable de dérivés dinitrés qu'il faut éliminer. On sait aussi préparer la nitro-2 tétrahydro-1,4,4a,9a anthraquinone par nitration de la tétrahydro-1,4,4a,9a anthraquinone (brevet japonais Kokaï 77.108.429). Toutefois cette méthode ne permet pas d'obtenir un dérivé mononitré susceptible de donner l'$\alpha$-nitro-anthraquinone. Il est connu en outre d'utiliser la tétrahydro-1,2,3,4 anthraquinone pour préparer ses dérivés nitro-5 et 6. (F 658.972)

La demanderesse a maintenant trouvé, et c'est là l'objet de la présente invention, qu'il est possible, par nitration de l'hexahydro-1,2,3,4,4a,9a anthracène-dione-9,10, d'obtenir sélectivement et avec d'excellents rendements les dérivés monosubstitués en position 5 et 6 de la tétrahydro-1,2,3,4 anthraquinone. On obtient la nitro-5 tétrahydro-1,2,3,4 anthraquinone et la nitro-6 tétrahydro-1,2,3,4 anthraquinone en proportions variables suivant les conditions de la réaction, la nitro-5 tétrahydro-1,2,3,4 anthraquinone étant le produit prépondérant.

L'hexahydro-1,2,3,4,4a,9a anthracène-dione-9,10 peut être préparée suivant le procédé de la demande de brevet français n° 78.19466 déposée le 29 Juin 1978 pour "Hexahydro-1,2,3,4,4a,9a anthracène-dione-9,10, sa préparation et son application à la délignification des matériaux lignocellulosiques" (inventeurs: Lucien BOURSON, Serge DELAVARENNE et Pierre TELLIER). Le procédé consiste essentiellement à soumettre à une hydrogénation puis à une isomérisation la tétrahydro-1,4,4a,9a anthraquinone, cette dernière résultant de la réaction de Diels Alder entre la naphtoquinone-1,4 et le butadiène.

La nitration de l'hexahydro-1,2,3,4,4a,9a anthracène-dione-9,10 peut être effectuée dans un mélange d'acide sulfurique et d'acide nitrique ou dans l'acide nitrique pur. Il est également possible d'opérer en présence d'un solvant inerte dans les conditions de la réaction. Dans le cas où la nitration est effectuée dans un mélange d'acide nitrique et d'acide sulfurique, la concentration de l'acide sulfurique est supérieure à 70% et de préférence supérieure à 90%. On peut aussi utiliser de l'oléum. La concentration de l'acide nitrique dépend de celle de l'acide sulfurique mais elle est de préférence supérieure à 90%. Dans le cas où la nitration est effectuée dans l'acide nitrique seul, la concentration de celui-ci est d'au moins 90% et de préférence au moins 98%.

La nitration est effectuée à une température comprise entre 0 et 50°C, de préférence entre 0 et 30°C.

Le nitro-5 tétrahydro-1,2,3,4 anthraquinone est beaucoup moins soluble que la nitro-6 tétrahydro-1,2,3,4 anthraquinone, aussi peut-elle être facilement isolée à l'état pur. Quand la nitration est effectuée dans un mélange d'acide sulfurique et d'acide nitrique, la nitro-5 tétrahydro-1,2,3,4 anthraquinone formée est insoluble dans le milieu et peut être isolée par simple filtration. De même à partir d'un mélange de nitro-5 tétrahydro-1,2,3,4 anthraquinone et de nitro-6 tétrahydro-1,2,3,4 anthraquinone, il est facile d'isoler la nitro-5 tétrahydro-1,2,3,4 anthraquinone pure par recristallisation dans un solvant approprié.

Les exemples suivants illustrent sans la limiter la présente invention.

## Exemple 1

Dans un réacteur de 250 ml muni d'un agitateur, contenant 98,4 g d'acide sulfurique à 96% et 18,2 g d'acide nitrique à 100%, on introduit 21,4 g d'hexahydro-1,2,3,4,4a,9a anthracène-dione-9,10 en 30 minutes à la température de 5°C. On maintient la température pendant 3 heures entre 15 et 18°C. On filtre. Le précipité lavé et séché pèse 16 g. Il est constitué de nitro-5 tétrahydro-1,2,3,4 anthraquinone pratiquement pure. Point de fusion: 184—185°C.

Spectre RMN (benzène deutérié à 60°C):

4 H méthylène en position 1 et 4 $\delta = 2,15$ ppm
4 H méthylène en position 2 et 3 $\delta = 1,18$ ppm
2 H aromatiques en position 7 et 8 $\delta = 6,93$ ppm
1 H aromatique en position 6 $\delta = 7,83$ ppm

Spectre IR:

$\gamma$ NO$_2$ à 1540 cm$^{-1}$
$\gamma$ CO à 1660 cm$^{-1}$

Par dilution du filtrat on recueille encore 6,7 g d'un produit constitué principalement par de la nitro-6 tétrahydro-1,2,3,4 anthraquinone.

2

Spectre RMN:

$$4\ H\ \text{méthylène en position 1 et 4}\ \delta = 2,25\ ppm$$
$$4\ H\ \text{méthylène en position 2 et 3}\ \delta = 1,22\ ppm$$
$$2\ H\ \text{aromatiques en position 7 et 8}\ \delta = 7,8\ ppm$$
$$1\ H\ \text{aromatique en position 5}\ \delta = 8,67\ ppm$$

Spectre IR:

$$\gamma\ NO_2\ \text{à 1530 cm}^{-1}$$
$$\gamma\ CO\ \ \text{à 1650 cm}^{-1}$$

Le rendement global est de 88,3%.

L'hexahydro-1,2,3,4,4a,9a anthracène-dione-9,10 peut être préparée comme suit conformément à l'exemple 1 de la demande de brevet français 78.19466:

"Dans un autoclave en acier inoxydable muni de dispositifs de chauffage et d'agitation, on introduit 100 ml de toluène, 21,2 g de tétrahydro-1,4,4a,9a anthraquinone et 0,2 g d'un catalyseur à base de palladium déposé sur charbon, contenant 5% de palladium. On chauffe à 100°C et introduit de l'hydrogène sous un pression de 30 bars. On poursuit la réaction pendant 4 heures en maintenant la pression entre 20 et 30 bars. Après refroidissement, on sépare par filtration 7 g d'un produit insoluble se présentant sous forme de cristaux verts fondant à 206—208°C de masse moléculaire 214, et dont les spectres RMN et IR montrent qu'il s'agit du tétrahydro-1,2,3,4 anthracène-diol-9,10. Par concentration du filtrat on obtient 14 g d'un produit fondant à 80—88°C (F: 89—91°C après recristallisation dans l'hexane) constitué par de l'hexahydro-1,2,3,4,4a,9a anthracène-dione-9,10 pratiquement pure."

### Exemple 2

Dans un réacteur analogue à celui de l'exemple 1, contenant 63 g d'acide nitrique à 100%, on introduit lentement 10,7 g d'hexahydro-1,2,3,4,4a,9a anthracène-dione-9,10. On maintient la température à 40°C pendant 6 heures, puis verse le mélange réactionnel dans un litre d'eau. Le précipité formé est lavé et séché. Il pèse 11 g; l'analyse par chromatographie en couche mince montre qu'il est constitué d'un mélange de nitro-5 tétrahydro-1,2,3,4 anthraquinone et de nitro-6 tétrahydro-1,2,3,4 anthraquinone. Le rendement est de 85,6%.

### Exemple 3

Dans un réacteur de 1 litre, muni d'un agitateur, contenant 630 g d'acide nitrique à 100%, on introduit lentement 107 g d'hexahydro-1,2,3,4,4a,9a anthracène-dione-9,10 de façon à ne pas dépasser 10°C. On maintient 6 heures à 10°C puis verse la solution obtenue dans 3 litres d'eau à 0°C. Le précipité formé est recueilli, lavé et séché. Il pèse 121 g; l'analyse par RMN montre qu'il contient 80% de nitro-5 tétrahydro-1,2,3,4 anthraquinone. Le rendement est de 94,2%. Par recristallisation de 110 g du précipité dans le perchloréthylène, on recueille 81 g de nitro-5 tétrahydro-1,2,3,4 anthraquinone pure. Point de fusion: 185°C.

**Revendications**

1. Procédé pour la préparation des dérivés mononitrés en position 5 ou 6 de la tétrahydro-1,2,3,4 anthraquinone caractérisé en ce que l'on soumet l'hexahydro-1,2,3,4,4a,9a anthracène-dione-9,10 à une nitration.

2. Procédé selon la revendication 1 caractérisé en ce que la nitration est effectuée dans un mélange d'acide nitrique et d'acide sulfurique.

3. Procédé selon la revendication 2 caractérisé en ce que la concentration de l'acide sulfurique est comprise entre 70 et 100%, de préférence supérieure à 90%.

4. Procédé selon la revendication 2 caractérisé en ce que la concentration de l'acide nitrique est comprise entre 70 et 100%, de préférence supérieure à 90%.

5. Procédé selon la revendication 1 caractérisé en ce que la nitration est effectuée dans l'acide nitrique pur.

**Patentansprüche**

1. Verfahren zur Herstellung von in 5- oder 6-Stellung mononitrierten Derivaten des 1.2.3.4-Tetrahydroanthrachinons, dadurch gegennzeichnet, daß man 1.2.3.4.4a.9a-Hexahydroanthrazen-1.10-dion einer Nitrierung unterwirft.

2. Verfahren gemäß Anspruch 1, dadurch gekennzeichnet, daß die Nitrierung in einem Gemisch aus Salpetersäure und Schwefelsäure durchgeführt wird.

3. Verfahren gemäß Anspruch 2, dadurch gekennzeichnet, daß die Konzentration der Schwefel-

**0 010 470**

säure zwischen 70 und 100%, vorzugsweise oberhalb 90% ist.

4. Verfahren gemäß Anspruch 2, dadurch gekennzeichnet, daß die Konzentration der Salpetersäure zwischen 70 und 100%, vorzugsweise oberhalb 90% ist.

5. Verfahren gemäß Anspruch 1, dadurch gekennzeichnet, daß die Nitrierung in reiner Salpetersäure durchgeführt wird.

## Claims

1. Process for the preparation of derivatives of 1,2,3,4-tetrahydro-anthraquinone, mononitrated in the 5 or 6 position, characterised in that 1,2,3,4,4a,9a-hexahydro anthracene 9,10 dione is subjected to nitration.

2. Process according to claim 1 characterised in that the nitration is effected in a mixture of nitric acid and sulphuric acid.

3. Process according to claim 2 characterised in that the concentration of the sulphuric acid is between 70% and 100%, preferably greater than 90%.

4. Process according to claim 2, characterised in that the concentration of the nitric acid is between 70% and 100%, preferably greater than 90%.

5. Process according to claim 1, characterised in that the nitration is effected in pure nitric acid.